# EUROPÄISCHE PATENTSCHRIFT

(11) **EP 0 619 303 B1**
(45) Veröffentlichungstag und Bekanntmachung des Hinweises auf die Patenterteilung: **13.08.1997**
(21) Anmeldenummer: 94104677.3
(22) Anmeldetag: 24.03.1994
(51) Int. Cl.: C07C 317/14, C07C 315/04, C07C 313/04, C07C 317/44

(54) **4-Alkyl-3-chlor-benzolsulfinsäuren,4-Alkyl-3-chlor-benzolsulfonylcarbonsäuren, 4-Alkyl-3-chlor-alkylsulfonylbenzole und Verfahren zu ihrer Herstellung**
4-alkyl-3-chloro-benzenesulfinic acids, 4-alkyl-3-chloro-benzenesulfonic acids, 4-alkyl-3-chloro-alkylsulfonylbenzenes and process for their preparation
Acides 4-alkyl-3-chloro-benzène sulfiniques, acides 4-alkyl-3-chloro-benzène sulfoniques, 4-alkyl-3-chloro-alkylsulfonylbenzènes et procédé pour leur préparation

(30) Priorität: 03.04.1993 DE 4311079
(43) Veröffentlichungstag der Anmeldung: 12.10.1994
(73) Patentinhaber: HOECHST AKTIENGESELLSCHAFT, 65929 Frankfurt am Main (DE)
(72) Erfinder: Folz, Georg, Dr., D-60529 Frankfurt (DE); Papenfuhs, Theodor, Dr., D-60433 Frankfurt (DE)

(56) Entgegenhaltungen:
- WO-A-90/06301
- WO-A-91/07384
- WO-A-92/14700
- US-A- 4 675 447
- HELVETICA CHIMICA ACTA, Bd.66, Nr.104, 1983 Seiten 1046 - 1052 A. COURTIN
- CHEMICAL ABSTRACTS, vol. 119, no. 1, 1993, Columbus, Ohio, US; abstract no. 8503n, & JP-A-05 025 119 (MITSUI TOATSU CHEMICALS) 2. Februar 1993
- CHEMICAL ABSTRACTS, vol. 119, no. 1, 1993, Columbus, Ohio, US; abstract no. 8503n, & JP-A-05 025 119

## Beschreibung

Verfahren zur Herstellung von 4-Alkyl-3-chlor-benzolsulfinsäuren, 4-Alkyl-3-chlor-benzolsulfonylcarbonsäuren, 4-Alkyl-3-chlor-alkylsulfonylbenzolen

Die Erfindung betrifft Verfahren zur Herstellung von 4-Alkyl-3-chlorbenzolsulfinsäuren, 4-Alkyl-3-chlor-benzolsulfonylcarbonsäuren, 4-Alkyl-3-chloralkylsulfonylbenzolen.

Gemäß der US-Patentschrift 4,675,447 soll die Umsetzung von 4-Alkylalkylsulfonylbenzolen mit Sulfurylchlorid in Gegenwart eines Katalysators zu 4-Alkyl-3-chlor-alkylsulfonylbenzolen führen. Ausgehend von 4-Methylmethylsulfonylbenzol ist jedoch lediglich die Herstellung von 4-Methyl-3-chlormethylsulfonylbenzol und damit auch nur die Existenz dieser Verbindung belegt. In der US Patentschrift 4,675,447 findet sich kein Hinweis auf die für die Herstellung von 4-Alkyl-3-chlor-alkylsulfonylbenzolen benötigten 4-Alkylalkylsulfonylbenzole - ausgenommen 4-Methyl-methylsulfonylbenzol. Nachteilig an diesem Verfahren ist der Einsatz von teurem Sulfurylchlorid, der zwangsweise zu einem erheblichen Anfall von umweltbelastendem Schwefeldioxid führt. Zudem erfordert das Verfahren die Bereitstellung eines möglichst trockenem Ausgangsmaterials.
Nachteilig ist ferner, daß aus der US Patentschrift 4,657,447 nicht hervorgeht, wie die als Ausgangsmaterial einzusetzenden 4-Alkyl-alkylsulfonylbenzole zu erhalten sind.

In WO-A-9214700 wird die Synthese von Alkylsulfonylalkylbenzolen aus den entsprechenden Sulfinaten mit Alkylhalogeniden beschrieben. Dies hat den Nachteil, daß gerade die niederen Alkylhalogenide (C₁-C₃) gasförmig sind, bzw. einen hohen Dampfdruck haben, und zum Teil giftig sind. Arbeiten unter Druck ist daher in diesen Fällen unumgänglich.
In WO-A-9006301 wird die Herstellung von 2-Chlor-4-methylsulfonyltoluol durch Chlorierung des 4-Methylsulfonyltoluols mit Sulfurylchlorid beschrieben. Diese Reaktion hat den Nachteil, daß stöchiometrische Mengen an SO₂ und HC entstehen. Zudem ist die Aufarbeitung wegen des nötigen Überschußes an Sulfurylchlorid ungünstig.

Es bestand der Bedarf ein Verfahren zu entwickeln, das die vorstehend genannten Nachteile vermeidet und zum einen von leicht zugänglichen Ausgangsmaterialien ausgeht und zum anderen eine Herstellung mit geringer Umweltbelastung ermöglicht und zudem die Möglichkeit eröffnet, die Alkylgruppen nach Wunsch in das Produkt einzufügen.

Diese Aufgabe wird gelöst durch ein Verfahren zur Herstellung von 4-Alkyl-3-chloralkylsulfonylbenzolen der Formel (1) 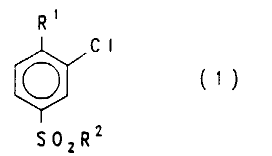 in welcher R¹, R² gleich oder verschieden sind und (C₁-C₄)-Alkyl bedeuten. Es besteht darin, daß man ein p-Alkylbenzolsulfonsäurechlorid der Formel (2) 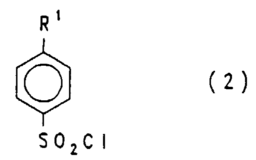 in welcher R¹ die vorstehend genannte Bedeutung hat, mit mindestens 1 Mol Chlor in Gegenwart eines Katalysators bei Temperaturen von etwa 50 bis etwa 100°C, zu einer Verbindung der allgemeinen Formel (3) 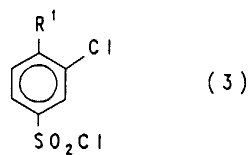 in welcher R¹ die vorstehend genannte Bedeutung hat chloriert, diese anschließend in wäßrigem Medium mit Natriumhydrogensulfit oder Natriumsulfit bei Temperaturen von etwa 40 bis etwa 90°C, zu einer Verbindung der allgemeinen Formel (4) 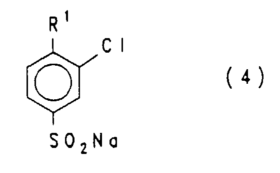 in welcher R¹ die vorstehend genannte Bedeutung hat, reduziert, diese mit einer α-Halogencarbonsäure der allgemeinen Formel (5) 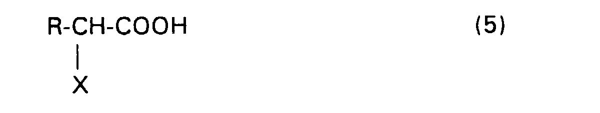 worin R Wasserstoff oder (C₁-C₃)-Alkyl und X Brom oder Chlor ist, oder einem ihrer Salze, zu 4-Alkyl-3-Chlor-4-benzolsulfonylcarbonsäuren der allgemeinen Formel (6), in welcher R¹ und R die vorstehend genannte Bedeutung haben, oder einem ihrer Salze 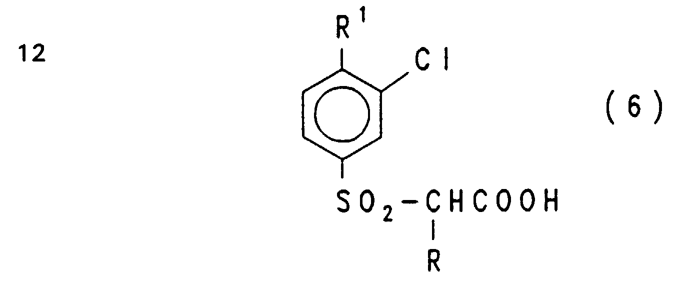 kondensiert und diese anschließend durch Erhitzen decarboxyliert.

Durch das erfindungsgemäße Verfahren können Verbindungen der Formel (1), in welcher R¹ gleich (C₁-C₄)-Alkyl und R² gleich (C₂-C₄)-Alkyl oder R¹ gleich (C₂-C₄)-Alkyl und R² gleich (C₁-C₄)-Alkyl bedeuten sowie Verbindungen der Formel (4), in welcher R¹ (C₁-C₄)-Alkyl bedeutet und Verbindungen der Formel (6) in welcher R Wasserstoff oder (C₁-C₃)-Alkyl und R¹ (C₁-C₄)-Alkyl bedeutet und ihre Alkalimetall-Salze erhalten werden.
Bevorzugt sind hierbei Verbindungen der Formel (1) in welcher R¹ gleich Methyl oder Ethyl und R² gleich Ethyl, Propyl oder Butyl, insbesondere Ethyl bedeuten. Diese Verbindungen sind wertvolle Ausgangsmaterialien für Herbicide, Insekticide und Pharmazeutika.

Das Verfahren geht aus von 4-Alkyl-benzolsulfonsäurechloriden, die nach bekannten Verfahren (Ullmanns Encyclopädie der technischen Chemie, Band 8, 4. Auflage, S. 422) aus Alkyl(C₁-C₄)-benzolen und Schwefelsäure mit anschließender Chlorierung erhalten werden können.
Die Chlorierung der 4-Alkylbenzolsulfonsäurechloride wird vorteilhaft in Substanz in Abwesenheit von Lösungsmitteln durch Einleiten von Chlor bei Temperaturen von etwa 50°C bis etwa 100°C in Gegenwart eines Chlorüberträgers als Katalysator durchgeführt.

In vielen Fällen hat es sich bewährt bei Temperaturen von 60 bis 90°C, vorzugsweise von 70 bis 80°C zu chlorieren. Als Katalysator (Chlorüberträger) hat sich Eisen(III)chlorid oder Eisen(III)-chlorid und Iod als vorteilhaft erwiesen. Es lassen sich jedoch auch z.B. SbCl₃ oder SbCl₅ als Katalysator verwenden. Die Chlorierung wird bei einem Chlorierungsgrad von ca. 100 % (Endpunktbestimmung durch Gaschromatographie) abgebrochen. Das Produkt enthält neben Spuren von Ausgangsverbindung nur sehr geringe Mengen der Dichlorverbindung. Es kann ohne Reinigung zur Weiterverarbeitung eingesetzt, aber auch gewaschen, sowie destilliert und fraktioniert werden. Die Ausbeute an 4-Alkyl(C₁-C₄)-3-chlor-benzolsulfonsäurechloriden ist nahezu quantitativ.

Die Reduktion der 4-Alkyl(C₁-C₄)-3-chlor-benzolsulfonsäurechloride zu den 4-Alkyl(C₁-C₄)-3-chlorbenzolsulfinsäuren kann mittels Natriumhydrogensulfit oder Natriumsulfit erfolgen (vgl. Houben-Weyl, Bd. 9, S. 305-311).

Vorteilhaft führt man die Reduktion in wäßrigem Medium durch, indem man eine Lösung von Natriumhydrogensulfit oder Natriumsulfit vorlegt und gleichzeitig 4-Alkyl-3-chlorbenzolsulfonsäurechlorid und 35 %ige Natronlauge unter pH-Kontrolle bei Einhaltung eines pH-Wertes von 8 bis 10 zutropft.

Man kann auch das Sulfonsäurechlorid zur Sulfitlösung geben und die Natronlauge unter Aufrechterhaltung eines pH von etwa 8 bis etwa 10 zugeben. Die Konzentration der Sulfitlösung kann zwischen etwa 10 und etwa 30 % liegen, wobei eine etwa 15 %ige Lösung zu bevorzugen ist. Die Sulfitmenge beträgt zwischen 1 bis 1,2 Mol, insbesondere 1,08 bis 1,15 Mol. Die Reaktion läuft bei einer Temperatur von 40 bis 90°C, bevorzugt zwischen 50 und 65°C ab. Die Reduktion ist beendet, wenn der pH-Wert von 8 bis 10 konstant bleibt. Aus der Lösung kristallisiert bei etwa 45°C das Natriumsalz der 4-Alkyl(C₁-C₄)-3-chlorbenzolsulfinsäure aus. Die Verbindung kann ohne Zwischenisolierung weiterverarbeitet werden, im selben Apparat, im Sinne eines Eintopfverfahrens.

Für die Alkylierung der erhaltenen Salze der 4-Alkyl-3-chlor-benzoslsulfinsäuren zu den 4-Alkyl-3-chlor-benzolsulfonylcarbonsäuren werden den Sulfinsäuren (Na-Salze) bei 40 bis 60°C die entsprechenden 2-Halogencarbonsäuren der allgemeinen Formel (5) bzw. deren Salze (Na, K, Ammonium) innerhalb einer Zeit von 10 Minuten bis 2 Stunden, bevorzugt in 0,5 h, zudosiert, in einer Menge von 1 bis 1,5 Mol, bevorzugt 1,1 bis 1,3 Mol. Im Falle der Zugabe der freien Carbonsäuren werden diese durch "Neutralisation" mit den entsprechenden Basen (z. B. Natronlauge 35 %ig) in die Salze überführt. Zur Komplettierung der Kondensation rührt man noch ca. 2 bis 3 Stunden bei 60°C nach (deutlich über 60°C sind Ausbeuteverluste durch verstärkte Hydrolyse der Halogencarbonsäure zu erwarten). Das in der Umsetzung gebildete Arylsulfonylcarbonsäurederivat kann isoliert werden, aber auch ohne Isolierung, durch Erhitzen der Reaktionslösung auf vorzugsweise 90 bis 110°C (Rückfluß) decarboxyliert werden, bis kein CO₂ mehr entweicht; Zeitbedarf 6 bis 12 Stunden. Das Produkt kann durch Kristallisation und Filtration, flüssig/flüssig-Trennung (Temperatur über dem Schmelzpunkt) und Extraktion (z. B. mit Toluol, Chlorbenzol etc.) erhalten werden.

Die nachstehenden Beispiele dienen zur Erläuterung der erfindungsgemäßen Verfahrens, ohne es darauf zu beschränken.

### Beispiel 1 (Chlorierung)

### Herstellung von 3-Chlor-4-methyl-benzolsulfonsäurechlorid

In einem Chlorierungsapparat werden 576 g (3 Mol) 4-Methylbenzolsulfonsäurechlorid aufgeschmolzen. Nach Zugabe von 7 g Eisen(III)-chlorid und 2 g Iod wird bei 75 bis 80°C so lange elementares Chlor eingeleitet
(5 l Cl₂/h), bis 100 %ige Chlorierung erfolgt ist (GC-Analyse).

Das Produkt kann nach Entfernen von Restchlor und HCl roh eingesetzt werden; es kann jedoch auch gewaschen und, wenn erwünscht, destilliert werden.

Die Ausbeute beträgt etwa 100 % d. Th. Reinheit ≥ 97 % (neben wenig Ausgangsverbindung und Dichlorverbindung).

### Beispiel 2

Herstellung von 3-Chlor-4-Methyl-methylsulfonylbenzol über die Natriumsalze von 3-Chlor-4-methylbenzolsulfinsäure (a) und 3-Chlor-4-methyl-benzolsulfonylessigsäure (b)
(a) Reduktion
   In einem mit 2 Tropftrichtern und einer pH-Elektrode versehenen Rührapparat werden 1800 ml Wasser und 420 g (3,3 Mol) Natriumsulfit vorgelegt und auf 50 bis 55°C erwärmt. Im Verlauf von etwa 2 Stunden läßt man 677 g (3 Mol) 3-Chlor-4-methyl-benzolsulfonsäurechlorid und gleichzeitig 690 g NaOH, 35 %ig so zulaufen, daß stets ein pH von 8 bis 10 gehalten wird. Die Temperatur darf dabei bis 65°C steigen. Die Reaktion ist beendet, wenn der pH-Wert von 8 bis 10 konstant bleibt. Die entstandene 3-Chlor-4-methyl-benzolsulfinsäure (Na-Salz) ist bei 65°C gelöst und kristallisiert ab ca. 55°C teilweise aus.
(b) Alkylierung
   Die Umsetzung des nach (a) gebildeten Sulfinats zum Methylsulfon erfolgt in derselben Apparatur. Dazu wird die unter (a) erhaltene Lösung bzw. Suspension bei 50 bis 60°C in einem Zeitraum von etwa 30 Minuten mit 331 g (3,5 Mol) Chloressigsäure versetzt. Mit 377 g Natronlauge, 35 %ig (3,3 Mol) wird auf pH 9 bis 10 gestellt (Bildung des Na-Salzes der Chloressigsäure) und zur Vervollständigung der Kondensation (Bildung des Natriumsazes der 3-Chlor-4-methyl-benzolsulfonylessigsäure) 2 bis 3 Stunden bei 60°C gerührt. Dann wird auf 95 bis 105°C (Rückfluß) erwärmt. Die Decarboxylierung setzt bei ca. 90°C allmählich ein und wird durch 6 bis 8-stündiges Erhitzen solange fortgeführt bis kein CO₂ mehr entweicht. Die zuerst klare Lösung geht in dem Maße, wie das Sulfons gebildet wird in eine Emulsion über, aus der bei Abkühlen das Produkt (3-Chlor-4-methyl-methylsulfonylbenzol) kristallin ausgeschieden und abfiltriert wird.
   Die Ausbeute beträgt etwa 90 %; Gehalt: ≥ 97 %; Schmelzpunkt 89 bis 91°C.

### Beispiel 3

### Herstellung von 3-Chlor-4-methyl-ethylsulfonylbenzol über die Natriumsalze der 3-Chlor-4-methylbenzolsulfinsäure (a) und 3-Chlor-4-methyl-benzolsulfonylpropionsäure (b)

Zu einer, gemäß Beispiel 2 (a) hergestellten Lösung bzw. Suspension von 3-Chlor-4-methylbenzolsulfinat werden in derselben Apparatur bei 50 bis 60°C innerhalb von ca. 30 Minuten 380 g (3,5 Mol) 2-Chlorpropionsäure eindosiert. Mit 380 g Natriumlauge, 35 %ig (3,3 Mol) wird auf pH 9 bis 10 gestellt (Bildung des Na-Salzes der Chlorpropionsäure) und zur Vervollständigung der Kondensation (Bildung des Na-salzes der 3-Chlor-4-methyl-benzolsulfonylpropionsäure) 2 bis 3 Stunden bei 60°C gerührt. Dann wird auf 95 bis 105°C (Rückfluß) hochgeheizt. Die Decarboxylierung setzt bei ca. 90°C allmählich ein und wird solange fortgesetzt bis kein CO₂ mehr entweicht. Durch Abkühlen wird auskristallisiert und das Produkt durch Filtration isoliert.

Die Ausbeute beträgt ca. 90 % d. Th., Gehalt: ≥ 97 %; Schmelzpunkt: 50 bis 52°C.
- C₉H₁₁ClO₂S: ber.:: C 49,38 %; H 5,029 %; Cl 16,23
gef.: C 49,19 %; H 5,014 %; Cl 16,37 %

### Beispiel 4 (Chlorierung)

### Herstellung von 3-Chlor-4-ethylbenzolsulfonsäurechlorid

Es wird, wie in Beispiel 1 beschrieben, gearbeitet, lediglich mit dem Unterschied, daß 615 g (3 Mol) 4-Ethylbenzolsulfonsäurechlorid (hergestellt durch z.B. Umsetzung von 4-Ethylbenzolsulfonsäure mit Chlorsulfonsäure) eingesetzt werden. Die Ausbeute beträgt etwa 100 % d. Th. Reinheit: ≥ 96 % (neben wenig Ausgangsverbindung und Dichlorverbindung)
Erstarrungspunkt: 21 bis 22°C
Siedepunkt (2,5 bis 3 mm Hg): 129 bis 130°C
- C₈H₈Cl₂O₂S: ber.:: C 40,18 %; H 3,37 %; Cl 29,65 %
gef.: C 40,02 %; H 3,29 %; Cl 29,73 %

### Beispiel 5

### Herstellung von 3-Chlor-4-ethyl-methylsulfonylbenzol über die Natriumsalze von 3-Chlor-4-ethylbenzolsulfinsäure (a) und 3 -Chlor-4-ethylbenzolsulfonylessigsäure (b)

(a) Reduktion
   Durchführung wie in Beispiel 2 (a) beschrieben, lediglich mit dem Unterschied, daß 718 g (3 Mol) 3-Chlor-4-ethylbenzolsulfonsäurechlorid eingesetzt werden. Das Natriumsalz der 3-Chlor-4-ethyl-benzolsulfinsäure kristallisiert erst bei tieferer Temperatur aus.
(b) Alkylierung
   Die Umsetzung des gebildeten Sulfinats zum Methylsulfon erfolgt in derselben Apparatur gemäß Beispiel 2 (b) über das Natriumsalz der 3 -Chlor-4-ethylbenzolsulfonylessigsäure.
   Die Ausbeute beträgt ca. 88 % d. Th.
   Gehalt: ≥ 96 %; Schmelzpunkt: 58 bis 60°C
   - C₉H₁₁ClO₂S: ber.:: C 49,427 %; H 5,07 %; Cl 16,21 %
   gef.: C 49,29 %; H 5,03 %; Cl 16,32 %

### Beispiel 6

Herstellung von 3-Chlor-4-ethyl-ethylsulfonylbenzol über die Natriumsalze von 3-Chlor-4-ethyl-benzolsulfinsäure (a) und 3-Chlor-4-ethylbenzolsulfonylpropionsäure (b)

Die Umsetzung einer gemäß Beispiel 5 (a) hergestellten Lösung bzw. Suspension von 3-Chlor-4-ethyl-benzolsulfinat (a) mit 2-Chlorpropionsäure zum Natriumsalz der 3-Chlor-4-ethyl-benzolsulfonylpropionsäure (b) und die Decarboxylierung erfolgt analog Beispiel 3. Alle Schritte werden in derselben Apparatur durchgeführt (Eintopfverfahren).

Die Ausbeute beträgt etwa 80 % d. Th.
Gehalt: ≥ 96 %; Schmelzpunkt: 36 bis 37°C
- C₁₀H₁₃ClO₂S: ber.:: C 51,609 %; H 5,633 %; Cl 15,233 %
gef.: C 51,53 %; H 5,49 %; Cl 15,37 %

## Patentansprüche

1. Verfahren zur Herstellung von 4-Alkyl-3-chloralkylsulfonylbenzolen der Formel (1) 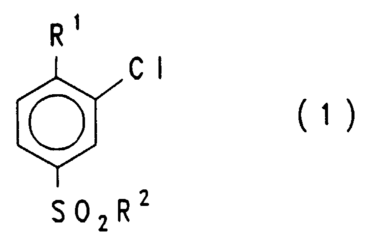 in welcher R¹, R² gleich oder verschieden sind und (C₁-C₄)-Alkyl bedeuten, indem man ein p-Alkylbenzolsulfonsäurechlorid der Formel (2) 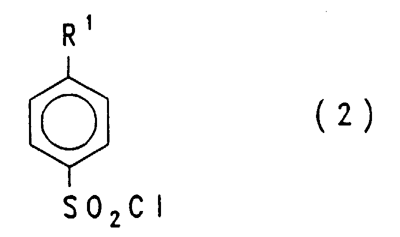 in welcher R¹ die vorstehend genannte Bedeutung hat, mit mindestens 1 Mol Chlor in Gegenwart eines Chlorüberträgers bei Temperaturen von etwa 50 bis etwa 100°C, zu einer Verbindung der allgemeinen Formel (3) 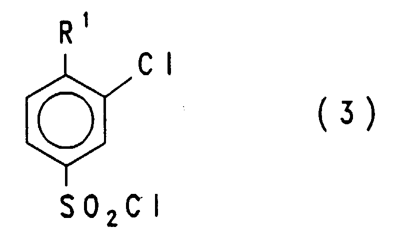 in welcher R¹ die vorstehend genannte Bedeutung hat chloriert, diese anschließend in wäßrigem Medium mit Natriumhydrogensulfit oder Natriumsulfit bei Temperaturen von etwa 40 bis etwa 90°C, zu einer Verbindung der allgemeinen Formel (4) 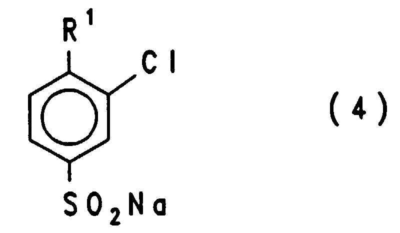 in welcher R¹ die vorstehend genannte Bedeutung hat, reduziert und diese mit einer α-Halogencarbonsäure der allgemeinen Formel (5) 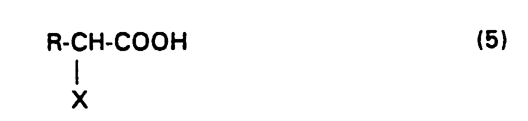 worin R Wasserstoff oder (C₁-C₃)-Alkyl und X Brom oder Chlor ist, oder einem ihrer Salze, zu einer 4-Alkyl-3-chlorbenzolsulfonylcarbonsäure der allgemeinen Formel (6), in welcher R¹ und R die vorstehend genannte Bedeutung haben, oder einem ihrer Salze 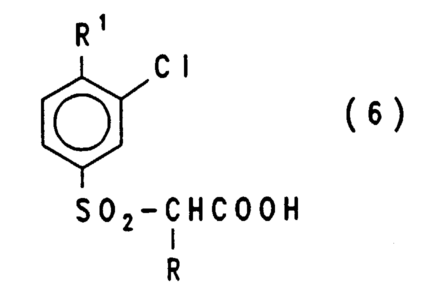 kondensiert und diese anschließend durch Erhitzen decarboxyliert.

2. Verfahren nach Anspruch 1, dadurch gekennzeichnet, daß man bei Temperaturen von 60 bis 90°C vorzugsweise 70 bis 80°C chloriert.

3. Verfahren nach Anspruch 1 oder 2, dadurch gekennzeichnet, daß als Chlorüberträger Eisen(III)-chlorid oder Eisen(III)-chlorid und lod verwendet wird.

4. Verfahren nach einem oder mehreren der Ansprüche 1 bis 3, dadurch gekennzeichnet, daß man mit Natriumhydrogensulfit oder Natriumsulfit bei Temperaturen von 40 bis 90°C, vorzugsweise 50 bis 65°C bei einem pH-Wert von 8 bis 10 reduziert.

5. Verfahren nach einem oder mehreren der Ansprüche 1 bis 4, dadurch gekennzeichnet, daß man 1 bis 1,2 Mol, vorzugsweise 1,08 bis 1,15 Mol Natriumhydrogensulfit oder Natriumsulfit einsetzt.

6. Verfahren nach einem oder mehreren der Ansprüche 1 bis 5, dadurch gekennzeichnet, daß man mit 1 bis 1,5 Mol, bevorzugt 1,1 bis 1,30 Mol einer α-Halogencarbonsäure oder einer ihrer Salze umsetzt.

7. Verfahren nach Anspruch 6, dadurch gekennzeichnet, daß die α-Halogencarbonsäure eine α-Chlorcarbonsäure ist.

8. Verfahren nach einem oder mehreren der Ansprüche 1 bis 7, dadurch gekennzeichnet, daß man mit α-Halogencarbonsäuren bei Temperaturen von 40 bis 70°C bevorzugt 50 bis 60°C umsetzt.

9. Verfahren nach einem oder mehreren der Ansprüche 1 bis 8, dadurch gekennzeichnet, daß es mit oder ohne Isolierung der Zwischenstufen durchgeführt wird.

## Claims

1. A process for preparing 4-alkyl-3-chloroalkylsulfonylbenzenes of the formula (1) 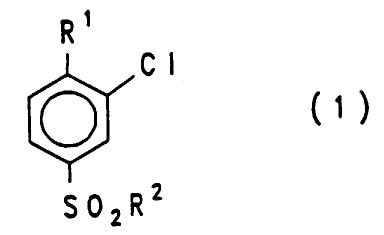 in which R¹ and R² are identical or different and are each (C₁-C₄)-alkyl, by chlorinating a p-alkylbenzenesulfonyl chloride of the formula (2) 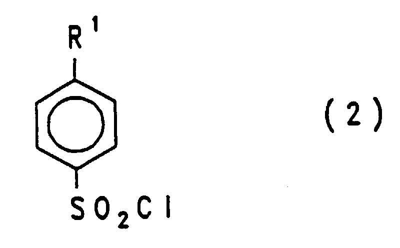 in which R¹ is as defined above, with at least 1 mol of chlorine in the presence of a chlorine transferrer at temperatures from about 50 to about 100°C to form a compound of the formula (3) 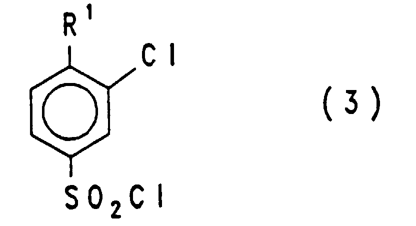 in which R¹ is as defined above, subsequently reducing this compound in aqueous medium with sodium bisulfite or sodium sulfite at temperatures from about 40 to about 90°C to a compound of the formula (4) 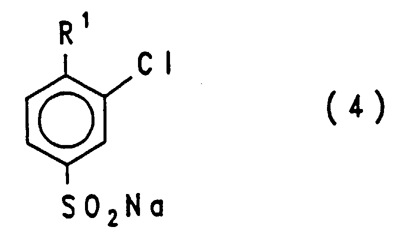 in which R¹ is as defined above, and condensing this compound with an α-halocarboxylic acid of the formula (5) 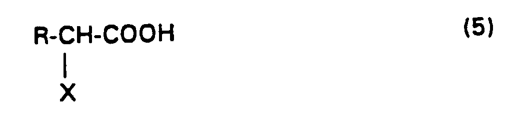 in which R is hydrogen or (C₁-C₃)-alkyl and X is bromine or chlorine, or a salt thereof, to form a 4-alkyl-3-chlorobenzenesulfonylcarboxylic acid of the formula (6), in which R¹ and R are as defined above, or a salt thereof 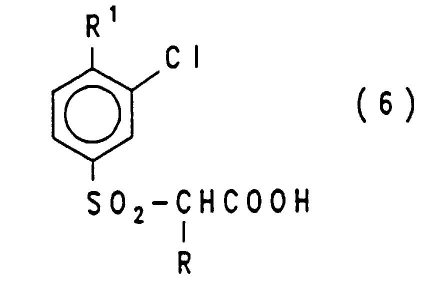 and subsequently decarboxylating it by heating.

2. The process as claimed in claim 1, wherein the chlorination is carried out at temperatures from 60 to 90°C, preferably from 70 to 80°C.

3. The process as claimed in claim 1 or 2, wherein the chlorine transferrer used is iron(III) chloride or iron(III) chloride and iodine.

4. The process as claimed in one or more of claims 1 to 3, wherein the reduction is carried out with sodium bisulfite or sodium sulfite at temperatures from 40 to 90°C, preferably from 50 to 65°C, at a pH from 8 to 10.

5. The process as claimed in one or more of claims 1 to 4, wherein from 1 to 1.2 mol, preferably from 1.08 to 1.15 mol, of sodium bisulfite or sodium sulfite are used.

6. The process as claimed in one or more of claims 1 to 5, wherein the reaction is carried out with from 1 to 1.5 mol, preferably from 1.1 to 1.30 mol, of an α-halocarboxylic acid or a salt thereof.

7. The process as claimed in claim 6, wherein the α-halocarboxylic acid is an α-chlorocarboxylic acid.

8. The process as claimed in one or more of claims 1 to 7, wherein the reaction is carried out with α-halocarboxylic acids at temperatures from 40 to 70°C, preferably from 50 to 60°C.

9. The process as claimed in one or more of claims 1 to 8, which is carried out with or without isolation of the intermediates.

## Revendications

1. Procédé pour la préparation des 4-alkyl-3-chloroalkylsulfonylbenzènes de formule (1) 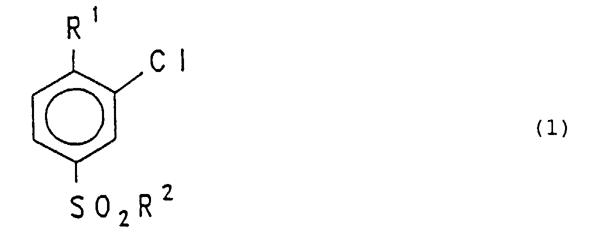 dans laquelle R¹, R² sont identiques ou différents et représentent alkyle en C₁-C₄, par chloration d'un chlorure d'acide p-alkylbenzènesulfonique de formule (2) 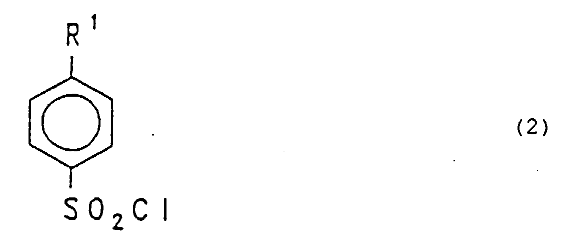 dans laquelle R¹ a la signification donnée précédemment, avec au moins un mole de chlore en présence d'un agent de transfert de chlore, à des températures d'environ 50 à environ 100 °C, pour obtenir un composé de formule générale (3) 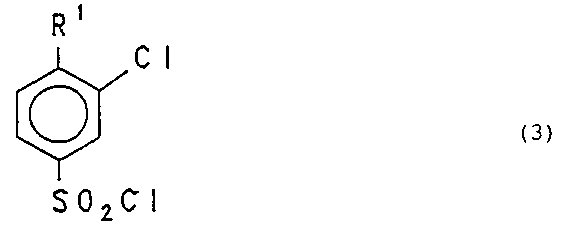 dans laquelle R¹ a la signification citée ci-dessus, on réduit ensuite celui-ci en milieu aqueux avec du bisulfite de sodium ou du sulfite de sodium, à des températures d'environ 40 à environ 90 °C, pour obtenir un composé de formule générale (4) 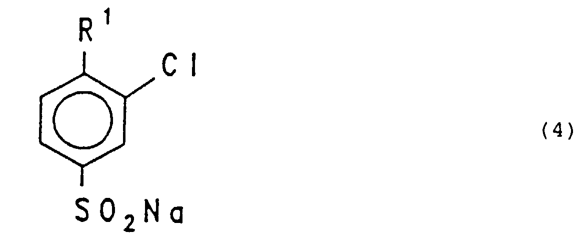 dans laquelle R¹ a la signification donnée ci-dessus, on condense celui-ci avec un acide a-halogénocarboxylique de formule générale (5) 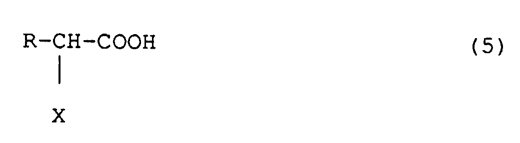 dans laquelle R représente l'hydrogène ou alkyle en C₁-C₃ et X représente le brome ou le chlore, ou un de ses sels pour obtenir un acide 4-alkyl-3-chloro-4-benzènesulfonylcarboxylique de formule générale (6) dans laquelle R¹ et R ont la signification donnée ci-dessus ou un de ses sels 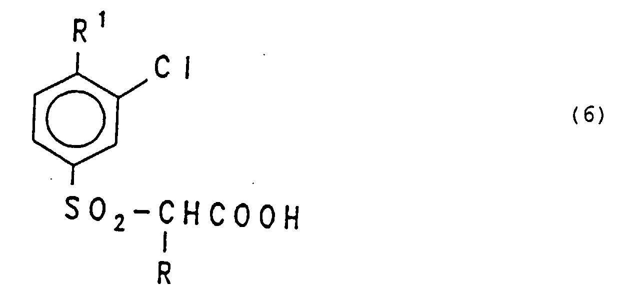 et ensuite on décarboxyle celui-ci par chauffage.

2. Procédé selon la revendication 1, caractérisé en ce que l'on effectue la chloration à des températures de 60 à 90 °C, de préférence de 70 à 80 °C.

3. Procédé selon la revendication 1 ou 2, caractérisé en ce que l'on utilise comme agent de transfert de chlore le chlorure de fer(III) ou le chlorure de fer(III) et l'iode.

4. Procédé selon une ou plusieurs des revendications 1 à 3, caractérisé en ce que l'on effectue la réduction à l'aide du bisulfite de sodium ou du sulfite de sodium à une température de 40 à 90 °C, de préférence de 50 à 65 °C à un pH de 8 à 10.

5. Procédé selon une ou plusieurs des revendications 1 à 4, caractérisé en ce que l'on utilise 1 à 1,2 mole, de préférence 1,08 à 1,15 mole de bisulfite de sodium ou de sulfite de sodium.

6. Procédé selon une ou plusieurs des revendications 1 à 5, caractérisé en ce que l'on effectue la réaction avec 1 à 1,5 mole, de préférence 1,1 à 1,30 mole d'un acide α-halogénocarboxylique ou de ses sels.

7. Procédé selon la revendication 6, caractérisé en ce que l'acide α-halogénocarboxylique est un acide α-chlorocarboxylique.

8. Procédé selon une ou plusieurs des revendications 1 à 7, caractérisé en ce que l'on effectue la réaction avec l'acide α-halogénocarboxylique à des températures de 40 à 70 °C, de préférence de 50 à 60 °C.

9. Procédé selon une ou plusieurs des revendications 1 à 8, caractérisé en ce qu'il est mis en oeuvre sans isolement des produits intermédiaires.
